(19) Europäisches Patentamt
European Patent Office
Office européen des brevets

(11) **EP 2 098 159 A1**

(12) **EUROPEAN PATENT APPLICATION**

(43) Date of publication:
**09.09.2009 Bulletin 2009/37**

(51) Int Cl.:
*A61B 1/005* [(2006.01)]  *G02B 23/24* [(2006.01)]

(21) Application number: **09003037.0**

(22) Date of filing: **03.03.2009**

(84) Designated Contracting States:
**AT BE BG CH CY CZ DE DK EE ES FI FR GB GR
HR HU IE IS IT LI LT LU LV MC MK MT NL NO PL
PT RO SE SI SK TR**
Designated Extension States:
**AL BA RS**

(30) Priority: **04.03.2008 JP 2008053361
06.03.2008 JP 2008055991**

(71) Applicant: **FUJIFILM Corporation
Minato-ku
Tokyo (JP)**

(72) Inventors:
• **Miyako, Kuniaki
Kanagawa (JP)**
• **Ashida, Tsuyoshi
Kanagawa (JP)**
• **Iida, Takayuki
Kanagawa (JP)**

(74) Representative: **Klunker . Schmitt-Nilson . Hirsch
Destouchesstrasse 68
80796 München (DE)**

(54) **Endoscope**

(57) An endoscope comprising: a bending mechanism for bending a bending portion; a manipulating section for bending the bending portion through the bending mechanism on the basis of a manipulative force applied by an operator; a driving unit that applies a driving force on the bending mechanism; resetting unit that inputs an instruction to reset a state where the bending portion is bent as a new neutral point; and control unit that controls the driving unit so as to maintain the state where the bending portion is bent corresponding to the neutral point reset on the basis of the instruction input by the resetting unit.

## Description

BACKGROUND OF THE INVENTION

The present invention relates to an endoscope for use in medical and other fields, and more specifically, to an endoscope having a bending portion provided near a distal end of an insertion section.

As is also well known, an endoscope includes an insertion section to be inserted into the human body, a manipulating section by which the insertion section is manipulated and the endoscope is manipulated for air insufflation/water feeding, a connector (LG-light guide-connector) that is connected to an air insufflation source, a suction pump, etc., and a universal cord (supply hose) for connecting the connector to the manipulating section and the insertion section.

As disclosed in JP 11-47082 A, JP 2000-229061 A, JP 2001-346756 A, and the like, a bending portion (angle portion), which can be bent in four directions, up and down and to the right and left, by turning a manipulative knob provided to the manipulating section, is typically provided near the distal end of the insertion section of the endoscope.

The bending portion is generally bent by being pulled with wires. Specifically, the bending portion has a configuration in which great number of rings is arrayed and linked in a tubular-form, where each ring is alternately linked to be swingable in the up or down direction and the left or right direction (two orthogonal directions). A total of four wires including a pair of wires spaced apart in the up down direction and a pair of wires spaced apart in the left and right direction, are inserted into the great number of rings, and the distal ends of such wires are fixed to the ring positioned on the most distal end side.

With respect to the manipulative knob, an UD (Up Down) knob for bending the bending portion to up or down, and an LR (Left Right) knob for bending the bending portion to left or right are provided to the manipulating section.

The basal ends of the two wires spaced apart in the up and down direction inserted to the rings of the bending portion is connected to each other to form one wire, and are wounded around a pulley that integrally rotates with the UD knob. Similarly, the basal ends of the two wires spaced apart in the left and right direction is connected to each other to form one wire, and are wounded around a pulley that integrally rotates with the LR knob.

Therefore, by turning the manipulative knob, one of the wires of each pair linked to the ring on the most distal end side of the bending portion is pulled while the other wire is advanced, whereby the bending portion can be bent. The bending portion can be bent to an arbitrary direction, up or down or to the right or left, by manipulating both the UD knob and the LR knob.

In the bending portion of the endoscope, the straight state in which the bending portion is not bent is normally set as the neutral point, which means the normal state.

Thus, when the manipulative knob is turned to bend the bending portion, the counter force acts so as to return the bending portion to the straight state, and the bending portion automatically returns to the straight state when the hand is released from the manipulative knob. Further, the counter force becomes larger as the amount of bending becomes larger, that is, greater force is required to further manipulate the bend (to turn the manipulative knob).

Therefore, the load on doctors, who are operators of the endoscope, becomes very large depending on the type of examinations to be conducted with the endoscope.

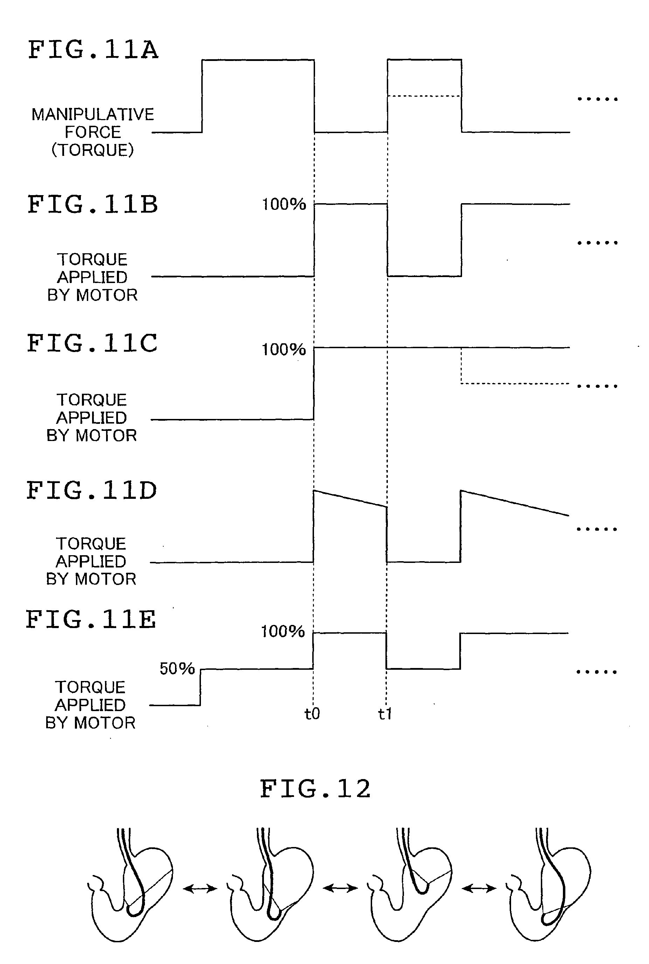

For instance, as conceptually illustrated in FIG. 12, the gastric fundus is examined by rotating the insertion section about its axis with the bending portion being greatly bent.

In other words, the doctor who manipulates the endoscope needs to hold the manipulating section having weight and rotate the insertion section while holding the manipulative knob so as not to return against the strong counter force, and thus the load becomes very large. If the doctor carelessly releases the hand from the manipulative knob, the bending portion returns to the straight state at once by the strong counter force, which may damage the human body as the target of examination.

In this regard, an endoscope equipped with a so-called brake mechanism for fixing the bending portion in the bent state has been proposed as described in JP 11-47082 A, JP 2000-229061 A, and JP 2001-346756 A.

The brake mechanism normally holds the bending portion in the bent state by stopping the turning of the manipulative knob with the frictional force, and puts the manipulative knob into a fixed state in which the manipulative knob does not move at all and a half-braking state in which the bent state of the bending portion is maintained but the angle (amount of bending) of the bending portion can be changed by turning the manipulative knob in accordance with the difference in the frictional force.

Therefore, the load of rotating the insertion section while holding the manipulative knob with the hand can be eliminated by using the brake mechanism. However, in order to adjust the angle of the bending portion in a half-brake applied state, the manipulative knob needs to be turned/manipulated with a force beyond the frictional force for maintaining the half-braking state, and thus force is required in the manipulation, and the load on the doctors is still large.

SUMMARY OF THE INVENTION

An object of the present invention is to solve the above-mentioned problem of the conventional technology, that is, for example, to provide an endoscope capable of greatly reducing the load of the operator and allowing easy and safe operation even when the rotation of the insertion section or the adjustment of the amount of bending need to be carried out with the bending portion greatly bent such as in the gastric fundus examination.

An endoscope according to a first aspect of the present invention includes an insertion section having a bending portion near its distal end, a state where the bending portion is not bent being set as a neutral point, the endoscope including: a bending mechanism for bending the bending portion; a manipulating section for bending the bending portion through the bending mechanism on the basis of a manipulative force applied by an operator; a driving means that applies a driving force on the bending mechanism; resetting means that inputs an instruction to reset a state where the bending portion is bent as a new neutral point; and control means that controls the driving means so as to maintain the state where the bending portion is bent corresponding to the neutral point reset on the basis of the instruction input by the resetting means.

An endoscope according to a second aspect of the present invention includes an insertion section having a bending portion near its distal end, a state where the bending portion is not bent being set as a neutral point, the endoscope including: a bending mechanism for bending the bending portion; a manipulating section for bending the bending portion through the bending mechanism on the basis of a manipulative force applied by an operator; a driving means that applies a driving force on the bending mechanism; a manipulative force detection means that detects a manipulative force applied on the manipulating section by the operator; and a control means that controls, when the manipulative force detected by the manipulative force detection means changes to be zero from a value other than zero, the driving means so as to apply to the bending mechanism a driving force corresponding to the value before the change.

BRIEF DESCRIPTION OF THE DRAWINGS

In the accompanying drawings:

FIG. 1 is a perspective view showing an entire structure of an endoscope according to a first embodiment of the present invention;
FIG. 2 is a partial cross-sectional view illustrating a manipulating section of the endoscope according to the first embodiment;
FIG. 3 is a partial cross-sectional view illustrating the manipulating section of the endoscope according to a modification example of the first embodiment;
FIG. 4 is a diagram conceptually illustrating the bending mechanism of the angle portion of the endoscope according to the first embodiment;
FIG. 5 is a plan view conceptually illustrating the angle portion of the endoscope according to the first embodiment;
FIG. 6A is a diagram describing the resetting of the neutral point of the angle portion in the endoscope according to the first embodiment;
FIGS. 6B and 6C are graphs for describing the manipulation when the neutral point of the angle portion is reset in the endoscope according to the first embodiment, respectively;
FIG. 7A is a diagram for describing a method of resetting the neutral point of the angle portion in the endoscope according to another modification example of the first embodiment;
FIG. 7B is a diagram conceptually illustrating the bending mechanism of the angle portion of the endoscope according to yet another modification example of the first embodiment;
FIGS. 8A and 8B are graphs for describing another example of the operation in the endoscope according to the first embodiment;
FIG. 9 is a perspective view illustrating an entire structure of an endoscope according to a second embodiment;
FIG. 10 is a view conceptually illustrating the bending mechanism of the angle portion in the endoscope according to the second embodiment;
FIG. 11A is a conceptual view for describing the manipulative force exerted on the manipulative knob of the endoscope according to the second embodiment;
FIGS. 11B to 11E are conceptual views for describing a pseudo-brake in the endoscope according to the second embodiment; and
FIG. 12 is a conceptual view for describing the gastric fundus examination by the endoscope.

DETAILED DESCRIPTION OF THE PREFERRED EMBODIMENTS

On the following pages, an endoscope of the present invention is described in detail with reference to the preferred embodiments illustrated in the accompanying drawings.

First Embodiment

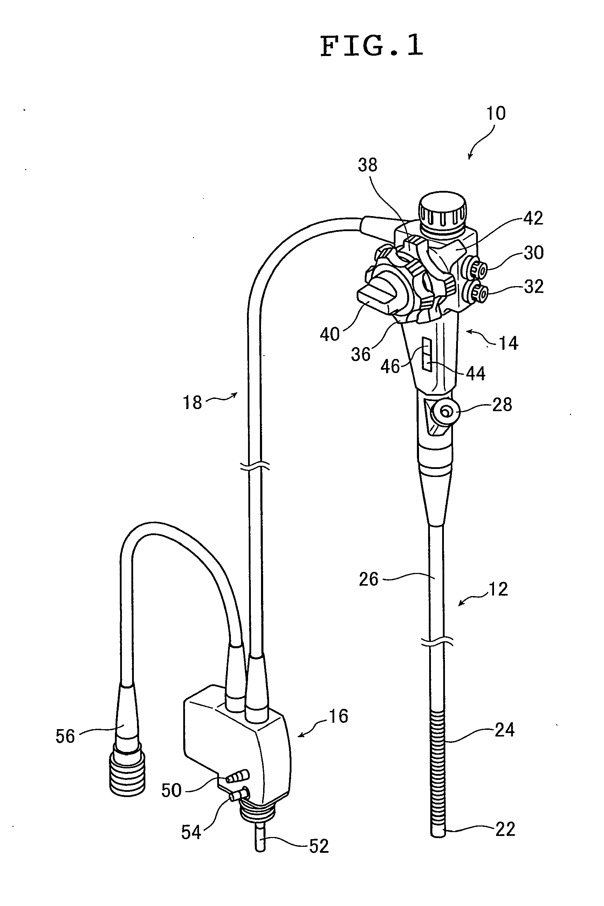

FIG. 1 is a view illustrating an entire structure of an endoscope 10 according to a first embodiment. The endoscope 10 is inserted into a part such as a body cavity (e.g. digestive tract or ears, nose, or larynx) to be examined and is used to observe the part, take a still or moving picture of the part, or even collect a tissue of the part.

The endoscope 10 is basically the same as a known conventional endoscope (endoscopic apparatus) other than that a neutral point resetting function allowing a neutral point of an angle portion 24 (stationary state in which bending is not manipulated with a manipulative knob) to be set to a desired bent state.

The endoscope 10 includes an insertion section 12, a manipulating section 14, a light guide (LG) connector 16, and a universal cord 18.

The insertion section 12 is an elongated part that is to be inserted into a part to be examined such as a body cavity, and has a distal end portion 22 at the distal end (distal end which is to be inserted and away from the manipulating section 14), the angle portion 24, and a flexible portion 26.

The distal end portion 22 is provided with a lighting glass for performing illumination with a light guide, air insufflation and water feeding nozzles through which air is typically withdrawn from the part under examination or air or water is fed to the part, and a forceps port through which forceps are inserted into the part under examination, typically to collect a tissue.

By way of example, the illustrated endoscope 10 is a so-called electronic scope for photographing the part under examination using an image sensor such as a CCD sensor, in which a photographing objective lens, a CCD sensor, a substrate for processing an image signal of an image photographed by the CCD sensor, and the like are attached to the distal end portion 22. The endoscope of the present invention is not limited to the electronic scope, and may be a so-called fiber scope with which the part under examination can be directly observed using an optical fiber. In this case, a viewing lens, a viewing window, and the like are attached to the distal end portion 22.

The angle portion (bending portion) 24 is a region which can be bent by manipulating the manipulating knobs (LR knob 36 and UD knob 38), to be described later, of the manipulating section 14, up and down or from right to left and vice versa (in two orthogonal directions) in order to insert the distal end portion 22 into a desired position or locate it in a desired position.

The flexible portion 26 is a part that connects the distal end portion 22 and the angle portion 24 to the manipulating section 14, and is an elongated member that is sufficiently flexible to allow the distal end portion 22 to be inserted into the part to be examined. The flexible portion 26 (and the angle portion 24) accommodates a forceps channel (tube) into which forceps are to be inserted, air insufflation and water feeding channels that are to be connected to the air insufflation and water feeding nozzles, a light guide for illuminating the part to be examined, and a cable for transferring an image (image signal) photographed by a CCD sensor.

The manipulating section 14 is a part for manipulating the endoscope 10. The manipulating section 14 is provided with a forceps port 28 through which forceps are to be inserted, a suction button 30 for performing suction through the air insufflation and water feeding nozzles at the distal end portion 22, and as well as an air insufflation/water feeding button 32 for performing air insufflation and water feeding through the air insufflation and water feeding nozzles at the distal end portion 22. If the endoscope 10 is an electronic scope, a variety of manipulating means for photographing the part to be examined such as a zooming switch and an imaging switch are attached to the manipulating section 14.

The manipulating section 14 is provided with manipulating knobs (bent manipulating means) for bending the angle portion 24 of the insertion section 12. Specifically, an LR knob 36 for bending the angle portion 24 to the left or right direction and a UD knob 38 for bending the angle portion 24 up and down in directions perpendicular to the above-mentioned left and right directions are provided in the manipulating section 14. In the endoscope 10, the LR knob 36 is turned to bend (flex) the angle portion 24 of the insertion section 12 to left or right direction whereas the UD knob 38 is turned to bend the angle portion 24 up and down (directions perpendicular to the right and left directions).

The manipulating section 14 is also provided with an LR brake manipulating section 40 which fixes the angle portion 24 being bent to the left or right direction by manipulating the LR knob 36, and a UD brake manipulating section 42 which fixes the angle portion 24 being bent up or down by manipulating the UD knob 38.

Furthermore, the manipulating section 14 is provided with a reset switch 44 for resetting the neutral point of the angle portion 24 from the normal straight state to the desired bent state, and a cancel switch 46 for returning the reset neutral point to the stationary straight state.

The configuration of the manipulative knob and the brake mechanism of the manipulating section 14, the configuration of the angle portion 24, the action of bending, and further, the reset switch 44 and the cancel switch 46 are described in more detail afterwards.

The LG connector 16 is a part that connects the endoscope 10 to water feeding means, air insufflation means, suction means, and the like installed in an endoscope processor (not shown), and the connector 16 is provided with a suction connector 50 for connecting the endoscope 10 to the suction means, a water feeding connector for connecting the endoscope 10 to the water feeding means, a ventilating connector for connecting the endoscope 10 to the air insufflation means, and the like.

The connector 16 is also provided with an LG rod 52 for connecting the illuminating light guide to a light source to be provided to the endoscope processor, and an S terminal 54 to which an S cord is connected to provide a path for diverting the leakage current to the endoscope 10 in the process of using an RF treating tool (so-called electrical scalpel such as electrical snare or knife).

The connector 16 is also connected with a video connector 56, which connects the endoscope 10 and a video processor for processing and displaying images, and the like photographed by the endoscope 10 (CCD sensor thereof).

The universal cord (LG flexible section) 18 is a part that connects the connector 16 to the manipulating section 14.

The lightguide, air insufflation and water feeding channels, etc. extending from the connector 16 pass through the universal cord 18 to be connected to the manipulating section 14, and the lightguide, air insufflation and water feeding channels, etc. extending from the manipulating section 14 pass through the flexible portion 26 of the insertion section 12 to be connected to the distal end portion 22 as described above.

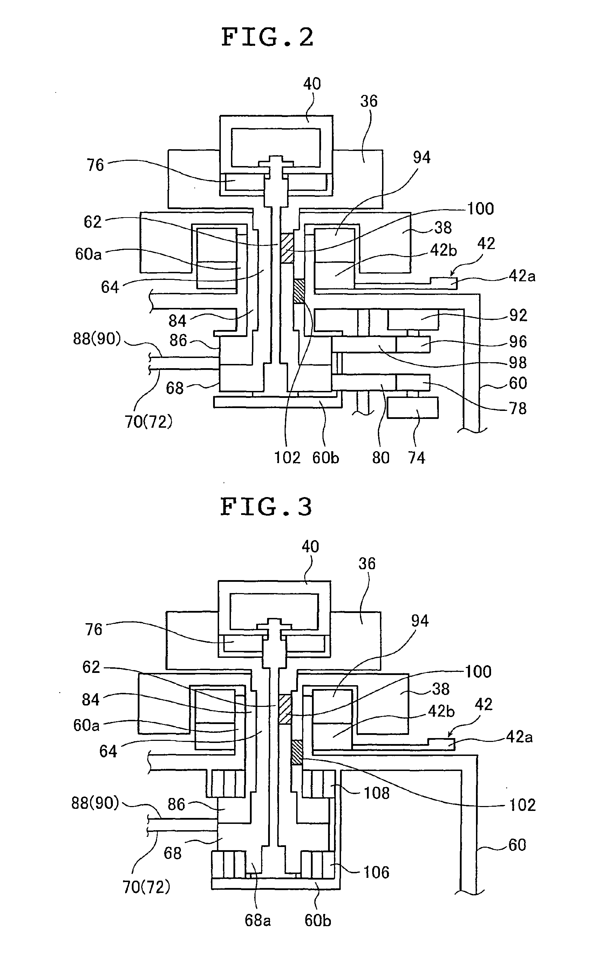

FIG. 2 illustrates a configuration of the LR knob 36, the UD knob 38, the LR brake manipulating portion 40, and the UD brake manipulating portion 42 in the manipulating section 14. The endoscope of the present invention is not limited to such a configuration, and all known configurations used in various types of endoscopes can be used.

A housing 60 of the manipulating section 14 has a cylindrical insertion portion 60a that is erected to penetrate the wall of the housing 60, and a substabtially C-shaped fixing portion 60b is provided at the lower end of the insertion portion 60a (in the inner side end portion of the housing 60).

The fixing portion 60b has a columnar central shaft 62 that is erected to pass through the insertion portion 60a to project outward from the housing 60.

A linking tube (rotating shaft) 64 is fixed to the underside of the LR knob 36 and a pulley 68 is fixed to the lower end of the linking tube 64. Two wires 70 and 72 for bending the angle portion 24 are connected to each other and wounded around the pulley 68 (alternatively, the ends of the wires 70 and 72 are respectively fixed to the pulley 68).

The wires 70 and 72 are adopted to bend the angle portion 24 to the left or right direction (angle wire/ manipulating wire), and are inserted to the angle portion 24 while being spaced apart from each other in the left and right direction by a wire guide and the like, the ends of the wires on the distal end portion 22 side being fixed to a distal end ring 112 of the angle portion 24, as described later.

In the pulley 68, there is also coaxially formed a gear that rotates by a LR motor 74 for resetting the neutral point in the left and right direction of the angle portion 24.

The linking tube 64 is a cylindrical member, through which the central shaft 62 passes, so as to be rotatably supported about the central shaft 62. Hence, both the LR knob 36 and the pulley 68 are also supported in a rotatable manner about the central shaft 62. When the LR knob 36 is turned by the operator, the pulley 68 is rotated by the same amount as that of the LR knob 36 so that any one of the wires 70 and 72 is pulled and the other wire is advanced.

The topside of the LR knob 36 is recessed to receive the lower part of the LR brake manipulating portion 40. Provided on the underside of the LR brake manipulating portion 40 is an LR brake member 76 for locking the LR knob 36 against turning.

The LR brake manipulating portion 40 is axially supported in a manner capable of turning with respect to the central shaft 62. On other hand, the LR brake member 76 is a cylindrical member, through which the central shaft 62 passes, so as to be supported by the central shaft 62 in such a manner that the LR brake member 76 cannot turn with respect to the central shaft 62 but can move along the length of the central shaft 62.

When the LR brake manipulating portion 40 is turned about the central shaft 62, the LR brake member 76 moves along the length of the central shaft 62 depending on the direction of turning by known means such as a cam mechanism or a screw mechanism.

As illustrated in FIG. 2, the LR brake member 76 is fully spaced apart from the LR knob 36 when being positioned on the LR brake manipulating portion 40 side. However, when the LR brake member 76 moves along the length of the central shaft 62 according to the turning of the LR brake manipulating portion 40, the LR brake member 76 is brought into contact with the LR knob 36 to be capable of depressing the LR knob 36. The LR brake member 76 is supported by the central shaft 62 to be incapable of turning, and hence, if the LR brake member 76 depresses the LR knob 36, frictional force develops to stop the turning of the LR knob 36, that is, put a brake on the LR knob 36. In addition, the LR brake member 76 does not turn with respect to the central shaft 62, and hence the above-mentioned braking action by no means causes the LR knob 36 to turn.

Note here that the LR brake manipulating portion 40 is adapted so as to be capable of adjusting, by controlling the amount of its turning, the force by which the LR brake member 76 depresses the LR knob 36, and the brake being exerted upon the LR knob 36 can take a fixed state or a half-braking state.

The fixed state is a state in which the LR brake member 76 depresses the LR knob 36 with such a great force that the LR knob 36 is incapable of turning. In contrast, the half-braking state is a state in which the LR knob 36 is sufficiently depressed with the LR brake member 76 to be prevented from automatic turning due to the counter force of the bent angle portion 24 but it is actually possible to turn the LR knob 36 though turning of the LR knob 36 requires an increased power due to the frictional force.

The LR motor 74 is fixed to the housing 60 of the manipulating section 14 by a stay (not shown) or the like, and a gear 78 is attached to the rotating shaft of the LR motor 74. A gear 80 is axially supported on the housing 60, and the gear 80 meshes with the gear 78 and the gear formed on the pulley 68.

Consequently, when the LR motor 74 is driven, the rotating force is transmitted to the pulley 68, thereby pulling one of the wires 70 and 72 and advancing the other wire, and bending the angle portion 24 to the left or right direction. In addition, the angle portion 24 can be held in the bent state by exerting torque on the pulley 68. Further, the LR knob 36 that is directly coupled to the pulley 68 also turns by driving the LR motor 74.

The manipulation of the LR knob 36 that is coupled to the pulley 68 through an intermediation of the linking tube 64, namely, the manipulation of the bending of the angle portion 24 to the left or right direction can be assisted.

The UD knob 38 is provided between the LR knob 36 and the housing 60. The UD knob 38 has a recess formed on its underside, in which the linking tube (rotating shaft) 84 is fixed to the ceiling of the recess and the pulley 86 is fixed to the lower end of the linking tube 84. The pulley 86 has two wires 88 and 90 wounded thereto, the wires being connected to the angle portion 24 so as to pull the angle portion 24 thereby to bend the same up and down (alternatively, the ends of the wires 88 and 90 are respectively fixed to the pulley 86). The wires 88 and 90 are provided to bend the angle portion 24 up and down, and are inserted to the angle portion 24 while being spaced apart in the up and down direction by the wire guide, the ends of the wires 88 and 90 on the distal end portion 22 side being fixed to the distal end ring 112 of the angle portion 24.

In the pulley 86, there is also coaxially formed a gear that rotates by a UD motor 92 for assisting the bending of the angle portion 24 in the up and down direction.

The linking tube 84 is a cylindrical member inserted into the insertion portion 60a of the housing 60, in which the linking tube 64 fixed to the LR knob 36 passes through the linking tube 84 such that the linking tube 84 is axially supported by the linking tube 64 in a rotatable manner. The pulley 86 at the lower end of the linking tube 84 is located on top of the pulley 68 at the lower end of the linking tube 64 fixed to the LR knob 36.

Hence, both the UD knob 38 and the pulley 86 are supported in a rotatable manner about the linking tube 64. When the UD knob 38 is turned by the operator, the pulley 86 is rotated by the same amount as that of the UD knob 38 so that one of the wires 88 and 90 is pulled and the other wire is advanced.

As described above, the linking tube 64 and the LR knob 36 rotate about the central shaft 62. As a result, the linking tube 84 and the UD knob 38 also rotate about the central shaft 62, and hence the LR knob 36 and the UD knob 38 that cause the angle portion 24 to bend rotate coaxially with each other.

The UD brake manipulating portion 42 is formed of a manipulating lever 42a and a cylindrical portion 42b. The cylindrical portion 42b is a cylindrical member through which the insertion portion 60a of the housing 60 passes in such a way that its upper part is inserted into the recess in the UD knob 38, and this cylindrical portion 42b is axially supported by the insertion portion 60a to be capable of rotation. The manipulating lever 42a has one end fixed to the cylindrical portion 42b and the other end projecting to the outside of the UD knob 38, and the cylindrical portion 42b can be rotated by manipulating the other end and swinging the manipulating lever 42a.

Provided on the upper side of the cylindrical portion 42b of the UD brake manipulating portion 42 is a UD brake member 94 for locking the UD knob 38 against turning. The UD brake member 94 is a cylindrical member that has the same inner and outer diameters as those of the cylindrical portion 42b and through which the insertion portion 60a of the housing 60 passes. Like the previously described LR brake member 76, the UD brake member 94 is supported by the insertion portion 60a so that the UD brake member 94 cannot be turned with respect to the insertion portion 60a but can move along the length of the insertion portion 60a.

When the UD brake manipulating portion 42 (cylindrical portion 42b) is turned about the insertion portion 60a, the UD brake member 94 moves along the length of the insertion portion 60a by known means such as a cam mechanism or a screw mechanism depending on the direction of turning.

As illustrated in FIG. 2, the UD brake member 94 is fully spaced apart from the UD knob 38 when being positioned on the cylindrical portion 42b side. However, when moving along the length of the insertion portion 60a depending on the turning of the UD brake manipulating portion 42, the UD brake member 94 is brought into contact with and depresses the UD knob 38, whereby frictional force develops to put a brake on the turning of the UD knob 38.

The UD brake manipulating portion 42 is adapted so as to be capable of adjusting, by controlling the amount of its turning, namely, the manipulating amount the manipulating lever 42a, the force by which the UD brake member 94 depresses the UD knob 38, and the brake being exerted upon the UD knob 38 can take a fixed state or a half-braking state.

The UD motor 92 is fixed to the housing 60 of the manipulating section 14, and a gear 96 is fixed to the rotating shaft of the UD motor 92. A gear 98 is axially supported on the housing 60, and the gear 98 meshes with the gear 96 and the gear formed on the pulley 86.

Consequently, when the UD motor 92 is driven, the rotating force is transmitted to the pulley 86, thereby pulling one of the wires 88 and 90 and advancing the other wire, and bending the angle portion 24 up or down. In addition, the angle portion 24 can be held in the bent state by exerting torque on the pulley 86.

In addition, the manipulation of the UD knob 38 that is coupled to the pulley 86 through an intermediation of the linking tube 84, namely, the manipulation of the bending up or down of the angle portion 24 can be assisted.

As illustrated in FIG. 2, the manipulating section 14 has a torque sensor 100 positioned in the hatched area of the linking tube 64 for sensing the torque exerted on the linking tube 64. The torque sensor 100 senses the manipulative force (rotational torque) exerted on the LR knob 36 because the LR knob 36, the linking tube 64, and the pulley 68 integrally rotate.

A torque sensor 102 is provided at a position in the hatched area of the linking tube 84 for sensing the torque exerted on the UD knob 38. The torque sensor 102 senses the manipulative force exerted on the UD knob 38 because the UD knob 38, the linking tube 84, and the pulley 86 integrally rotate.

As described later in detail, in the endoscope 10 of the illustrated example, the manipulative force exerted on the manipulative knobs 36, 38 is sensed in response to the input instruction from the reset switch 44 at the time, and the motors 74, 92 are driven (motor exerts torque on the pulley) so that the pulleys 68, 86 rotate at the same force as the manipulative force to thereby reset the neutral point of the angle portion 24.

In other words, in the manipulating section 14 of the illustrated example, the torque sensor 100, 102 is arranged at part of the cylindrical linking tube 64, 84 which is directly coupled to the manipulative knob 36, 38 and which rotates integrally with that manipulative knob 36, 38, and hence the manipulative force exerted on the manipulative knob 36, 38 to bend the angle portion 24 is directly sensed.

Various known types of torque sensors including a torque sensor that uses a strain gage and a magnetostrictive torque sensor may be utilized as the torque sensors 100, 102.

In the present invention, the manipulative force for bending the angle portion 24 is not limited to being sensed at the linking tubes 64, 84, and the manipulative force for bending the angle portion 24 may be directly or indirectly sensed at various positions and parts by, for example, sensing of torque at the pulleys 68, 86 or sensing of torque at the gears 80, 98. Further, various types of force sensing means other than the torque sensor may be utilized for the manipulative force sensing means.

In the case illustrated in FIG. 2, the rpm caused by the motors 74 and 92 for maintaining the bent state of the angle portion 24 is reduced by the gear, but this is not the sole case of the present invention, and a planetary gear or a harmonic drive may be provided to reduce the rpm caused by the motors 74 and 92. Alternatively, this mechanism may be combined with a gear to reduce the rotational speed.

Instead of applying the rotational force on the pulleys 68, 86 by reducing the rpm of the motors 74, 92, the pulleys 68, 86 may be directly rotated using a direct drive motor (DD motor).

For example, as illustrated in FIG. 3, a DD motor with an inner rotor is used as an LR motor 106 whose rotor is in engagement with the cylindrical portion 68a formed at the lower part of the pulley 68 for bending the angle portion 24 to right or left direction. The torque is directly applied on the pulley 68 by the LR motor 106.

Similarly, a DD motor with an inner rotor is used as a UD motor 108 whose rotor is in engagement through passing with the linking tube 84 linked to the pulley 86 for bending the angle portion 24 up or down. The torque is directly applied on the pulley 86 by the UD motor 108.

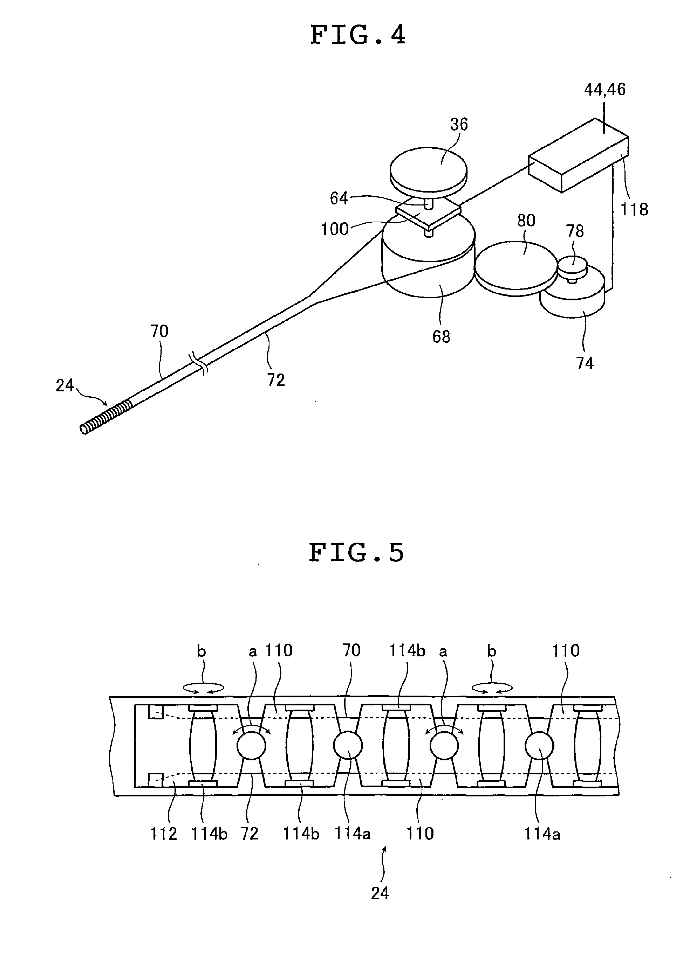

FIG. 4 conceptually illustrates the configuration of a mechanism by which the LR knob 36 is turned to bend the angle portion 24 to the left or right direction in the case illustrated in FIG. 2.

The angle portion 24 is similar to the angle portion of a known endoscope in that the angle portion 24 has a number of circular rings connected together, and is bent to the left or right direction by the wires 70 and 72 manipulated by the LR knob 36. The angle portion 24 is also bent up or down by the wires 88 and 90 manipulated by the UD knob 38 (not shown in FIG. 4).

FIG. 5 is a plan view illustrating a schematic construction of the angle portion 24.

Note that the construction of the angle portion 24 in the endoscope of the present invention is by no means limited to the illustrated case and that all designs that are adopted in a variety of endoscopes can be utilized.

In the illustrated case, the angle portion 24 includes a total of nine rings connected together, which consist of eight circular rings 110 and one distal end ring 112.

Each circular ring 110 has a shape as if a ring-shaped member is slightly curved at one diameter portion, in which both surfaces in the axial direction are formed by a convex surface and a concave surface, respectively. The distal end ring 112 is a substantially cylindrical member, and is arranged at the most distal end portion 22 side of the angle portion 24.

The circular rings 110 and the distal end ring 112 are linked to each other by linking pins 114a and linking pins 114b.

Eight circular rings 110 are arranged in such a way that their curving directions (directions of curved convex and concave) alternate along the length of the insertion section 12. Each linking pin 114a circularly links the convex portions of the convex surfaces of the pair of circular rings 110 facing each other in such a way that the circular rings 110 can rotate (swing) from right to left and vice versa (in the direction indicated by two-headed arrow a). In contrast, each linking pin 114b links the concave portions of the concave surfaces of the pair of circular rings 110 facing each other in such a way that the circular rings 110 can rotate (swing) up and down (in the direction indicated by two-headed arrow b). Similarly, the distal end ring 112 and the circular ring 110 facing thereto are also linked in such a way that the distal end ring 112 and the circular ring 110 can rotate (swing) up and down (in the direction indicated by two-headed arrow b) by the pair of linking pins 114b.

The linking pins 114a and the linking pins 114b are alternately arranged, and a plurality of circular rings 110 are linked so as to be capable of turning in two alternate directions, one being vertical and other horizontal.

The wires 70 and 72 for bending the angle portion 24 to the left or right direction are guided by the wire guide (not shown), and spaced apart in the horizontal direction (vertical to the paper on which FIG. 5 is drawn) to pass through the plurality of circular rings 110. The distal end of the wire 70 is secured to the inner right side of the distal end ring 112 whereas the distal end of the wire 72 is secured to the inner left side of the distal end ring 112.

Though not illustrated in FIG. 5, the wires 88 and 90 for bending the angle portion 24 up and down are guided by the wire guide, and spaced apart in the vertical direction (perpendicular to the paper on which FIG. 5 is drawn) to pass through the circular rings 110. The distal end of the wire 88 is secured to the inner upper side of the distal end ring 112 whereas the distal end of the wire 90 is secured to the inner lower side of the distal end ring 112.

As described above, the pulley 68 is fixed to the LR knob 36 through an intermediation of the linking tube 64, and the wires 70 and 72 are wounded around the pulley 68.

Therefore, the linking tube 64 and the pulley 68 rotate by turning the LR knob 36, and one of the wires 70 and 72 is pulled and the other wire is advanced depending on the direction of rotation of the pulley 68.

The plurality of circular rings 110 of the angle portion 24 are linked in such a way that the circular rings 110 can turn to the left or the right, or up and down alternately by the linking pins 114a and 114b, and hence when the pulley 68 is rotated through the LR knob 36, the angle portion 24 is bent to the left or the right with the pulled wire being on the inner side.

The greater the amount by which the wire 70 or 72 is pulled, namely, the greater the amount of rotation of the pulley 68, the greater the degree by which the angle portion 24 is bent. Therefore, the angle (amount of bending) of the angle portion 24 can be adjusted by controlling the amount by which the LR knob 36 is turned.

When bent, the angle portion 24 attempts to return to a linear state in which it is not bent, or a straight state, by the counter force it generates, and automatically returns to the straight state by such counter force when the hand is released from the LR knob 36. The counter force of the angle portion 24 is stronger the larger the angle. Therefore, a large force is required to bend the angle portion 24 the larger the angle.

Similarly, the pulley 86 is fixed to the UD knob 38 through an intermediation of the linking tube 84, and the wires 88 and 90 are wounded to the pulley 86. When the UD knob 38 is turned, either of the wires 88 or 90 is pulled, and the other wire is advanced depending on the direction of rotation of the pulley 86, and the angle portion 24 is bent up or down with the pulled wire on the inner side.

The angle of the angle portion 24 bent upward or downward can be adjusted by controlling the amount by which the UD knob 38 is turned. The counter force to return to the straight state is stronger the larger the angle with respect to the bend upward or downward, and a large force is required to bend the angle portion 24.

Therefore, by manipulating the LR knob 36 and the UD knob 38, the doctor who is the operator of the endoscope can bend the angle portion 24 by a desired angle (less than or equal to the limit of bending) up or down, to the left or the right, and in any direction that is a combination of the up or down direction and the right or left direction, whereby the operator can observe or take a picture of the part under examination, or collect a tissue from that part.

Note that the mechanism by which the angle portion of the endoscope is bent is by no means limited to the illustrated case and one may utilize various means (mechanisms) for bending the angle portion adopted in various types of endoscopes.

As described above, the rotating shaft of the LR motor 74 is coupled to the pulley 68 through an intermediation of the gears 80 and 78, and the wires 70 and 72 are moved back to bend the angle portion 24 to the left or the right by rotating the pulley 68 by means of the LR motor 74.

Similarly, the rotating shaft of the UD motor 92 is coupled to the pulley 86 by way of the gears 98 and 96, where the wires 88 and 90 are moved back to bend the angle portion 24 up or down by rotating the pulley 86 by means of the UD motor 92.

Here, the linking tube 64 that is linked to the pulley 68 is provided with the torque sensor 100, and the linking tube 84 that is linked to the pulley 86 is provided with the torque sensor 102, and the detection results of the torque sensors 100 and 102 are input to a control means 118, shown in FIG. 4, for controlling the drive of the LR motor 74 and the UD motor 92.

A signal corresponding to the input instruction of the reset switch 44 and the cancel switch 46 is also supplied to the control means 118. The reset switch 44 is a switch for resetting the neutral point of the angle portion 24 from the normal straight state to the desired bent state. On the other hand, the cancel switch 4 6 is a switch for returning the reset neutral point to the stationary straight state.

The operation of the control means 118 is described below taking the bend of the angle portion 24 to the left or the right through the manipulation of the LR knob 36 by way of example.

When the reset switch 44 is operated, the control means 118 input the sensed value of the torque sensor 100 and drives the LR motor 74 so that a torque of the same amount as the sensed value is exerted on the pulley 68. The LR motor 74 thus exerts on the pulley 68, that is, on the linking tube 64 a torque of the same amount as the torque exerted on the linking tube 64 at the point the reset switch 44 is operated.

The angle portion 24 can be maintained in the bent state by driving the LR motor 74 such that the torque of the same amount as the manipulative force at which the LR knob 36 is turned in order to bend the angle portion 24, that is, the torque exerted on the linking tube 64 is exerted on the pulley 68. In other words, the counter force of the angle portion 24 and the torque exerted by the LR motor 74 are balanced to each other, and hence the angle portion 24 can be maintained in the same bent state as when the reset switch 44 is operated.

Further, the counter force of the angle portion 24 generated by the bending of when the reset switch 44 is operated and the torque exerted on the pulley 68 by the LR motor 74 are in an equilibrium state. Therefore, if the LR knob 36 is further turned from such equilibrium state in a direction of bending the angle portion 24, the angle portion 24 attempts to return to the reset neutral point by the counter force that increased by the increase in angle, whereas if the LR knob 36 is turned in a direction of returning the bending of the angle portion 24, the counter force generates from the torque exerted on the pulley 68 from the LR motor 74 and the angle portion 24 attempts to return to the reset neutral point.

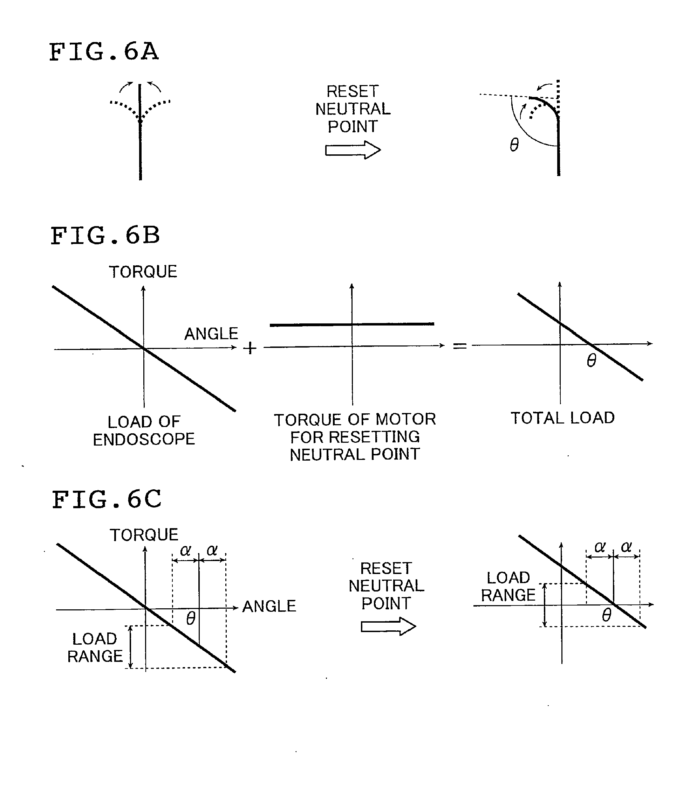

As conceptually illustrated in FIG. 6A, the neutral point of the angle portion 24 normally in the straight state can be reset to a bent state.

The relationship between the angle and the torque for bending (load by endoscope) is in a state illustrated on the left side of FIG. 6B at the neutral point of the normal straight state. If the torque is exerted by the LR motor 74 (middle of FIG. 6B) in order to reset the state of angle θ of the angle portion 24 as the neutral point, both torques are added, and the relationship between the angle and the torque (total load on the manipulation of the LR knob 36) becomes a state illustrated on the right side of FIG. 6B. In other words, the load shifts upward by the torque from the LR motor 74.

Therefore, when changing the angle of the angle portion 24 from the state of angle θ by an angle $\pm\alpha$, the manipulation needs to be carried out in a range of large load, as illustrated on the left side of FIG. 6C, in a state the neutral point is not reset. If the neutral point is reset at the angle θ, on the other hand, the manipulation can be carried out, as illustrated on the right side of FIG. 6C, in a range of small load (same load as manipulation at the neutral point of normal straight state), and the load of the doctor can be reduced. In particular, greater force is required for the manipulation as the angle becomes larger, as described above, the manipulative force can be greatly reduced when manipulating in the direction of increasing the amount of bending.

Therefore, according to the endoscope of the present invention, the insertion section can be rotated with the hand being released from the manipulative knob by resetting the neutral point even when the insertion section needs to be rotated with the bending portion being greatly bent such as in the gastric fundus examination. Further, unlike the state in which the half-brake is applied, manipulation can be carried out with a small force similarly to the manipulation at the neutral point of the normal straight state even when adjusting the angle from the reset neutral point.

The angle portion 24 can be held in a bent state by resetting the neutral point. Therefore, it is possible to prevent accidents such as damaging the human body by the angle portion 24 suddenly returned to the straight state by the counter force that occur when the hand is carelessly released from the manipulative knob with the angle portion 24 being in the bent state.

The manipulating section 14 is also provided with the cancel switch 46 for canceling the once reset neutral point, and returning the neutral point to the normal straight state. When the cancel switch 46 is operated, the control means 118 stops driving the LR motor 74 and releases the torque exerted on the pulley 68. The neutral point of the angle portion 24 then returns to the normal straight state.

Preferably, the control means 118 gradually reduces the output of the LR motor 74, that is, the torque exerted on the pulley 68 from the LR motor 74 and stops the drive of the LR motor 74 so that the angle portion 24 gradually returns to the straight state instead of immediately stopping the drive of the LR motor 74 in response to the signal from the cancel switch 46. As a result, the damage of the human body and the like that occurs when the angle portion 24 suddenly returns to the straight state can be prevented.

In the first embodiment described above, the torque sensor 100 senses the torque exerted on the linking tube 64 (manipulative force on the LR knob 36) in response to an instruction input by the reset switch 44, and the LR motor 74 is driven to exert the torque of the amount same as the sensed torque on the pulley 68 to reset the neutral point of the angle portion 24. However, this is not the sole case of the present invention, and the neutral point of the angle portion 24 can be reset with various types of means.

By way of example, using the relationship between the displacement of the bending means by the manipulation of the manipulative knob and the force necessary for bending at the angle the displacement is produced, the force necessary for resetting the neutral point may be calculated from the displacement at the point the resetting of the neutral point of the angle portion 24 is instructed, and the angle portion 24 may be bent with the necessary force to reset the neutral point of the angle portion 24.

In FIG. 4, when the angle portion 24 is bent by manipulating the LR knob 36, the position of a predetermined point of the wire 70 (or wire 72) changes depending on the angle of the angle portion 24, and the pulley 68 rotates thereby changing the rotating position.

The relationship between the angle of the angle portion 24 and the manipulative force necessary for realizing the angle such as the torque that needs to be exerted on the linking tube 64 (pulley 68 and LR knob 36) by turning the LR knob 36 can be known in advance.

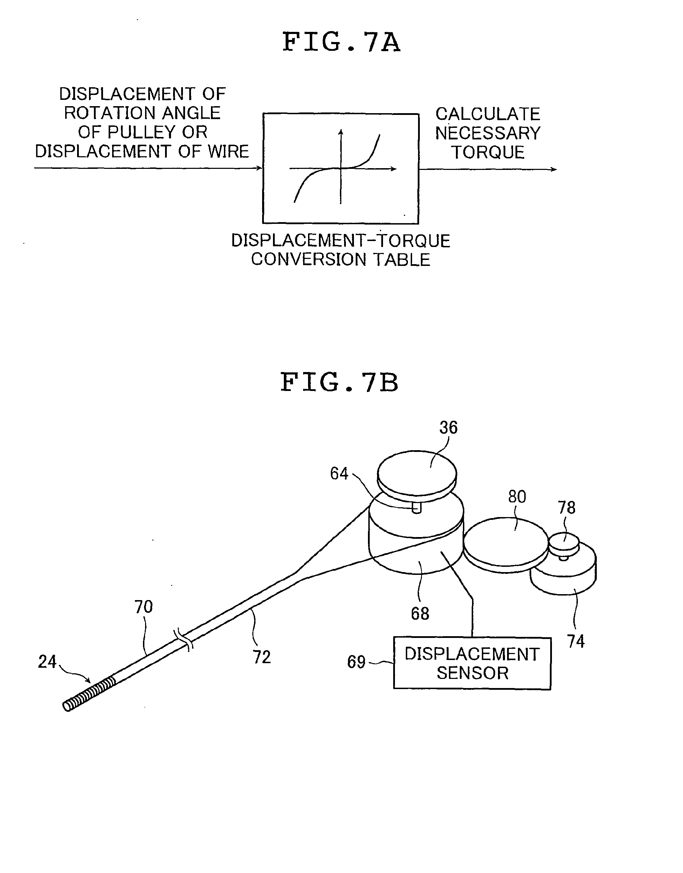

Therefore, as illustrated in FIG. 7A, a displacement-torque conversion table illustrating the relationship between the amount of displacement from the manipulation of the LR knob 36 to bend the angle portion 24 (wire position or pulley rotating position) and the torque exerted on the linking tube 64 in correspondence to the amount of displacement is formed in advance, and stored beforehand in, for example, the memory of the control means 118.

In other words, the displacement-torque conversion table is a table illustrating the relationship between the displacement of the wire or the pulley when a certain angle is obtained, and the torque necessary for realizing such angle.

In this arrangement, as shown in FIG. 7B, a displacement sensor 69 for sensing the rotational displacement of the pulley 68 or a displacement sensor for sensing the displacement of the wire 70 or 72 is provided in place of the torque sensor 100. Known displacement sensors can be used for such displacement sensors.

When the reset switch 44 instructs the resetting of the neutral point of the angle portion 24, the control means 118 inputs the displacement corresponding to the bending of the angle portion 24 at the time sensed by the displacement sensor, and calculates the torque exerted on the pulley 68 in correspondence to the displacement using the displacement-torque conversion table. In other words, the torque (necessary torque) necessary for realizing the angle that causes such displacement is calculated.

The control means 118 then drives the LR motor 74 such that the calculated necessary torque is exerted on the pulley 68. As a result, the necessary torque is then exerted on the pulley 68, and the angle portion 24 is maintained at the bent state, namely, the neutral point of the angle portion 24 is reset.

As a method of resetting the neutral point in the present invention other than the above, the neutral point of the angle portion 24 may be reset to the input instructed angle.

With reference to FIG. 4, the relationship between the angle of the angle portion 24 and the manipulative force necessary for realizing such angle, such as the torque that needs to be exerted on the pulley 68 by turning the LR knob 36 is known in advance.

The relationship between the angle and the torque that needs to be exerted on the pulley 68 is found and tabulated for various angles, and an input function of the angle such as 60°, 90°, and 180° is provided to the reset switch 44 in addition to the function of instructing the reset of the neutral point. Alternatively, the angle may be arbitrarily set using a dial and the like.

When the resetting of the neutral point and the angle are instructed (input), the control means 118 reads out the torque necessary for the instructed angle from the table, and drives the LR motor 74 such that the torque necessary for realizing such angle is exerted on the pulley 68 to thereby reset the neutral point of the angle portion 24.

The resetting mechanism of the neutral point having the input means of the angle may be simultaneously used with a mechanism of sensing the torque exerted on the linking tube 64 and resetting the neutral point of the angle portion 24 according to the sensed result, and a mechanism of sensing the displacement of the bending means and resetting the neutral point of the angle portion 24 according to the sensed result.

The description above is made on an example of resetting and canceling the neutral point in the left and right direction, which correspond to the manipulation of the LR knob 36, but the resetting of the neutral point and the canceling of the reset neutral point are carried out on the bend in the up and down direction by the UD knob 38 in the exact same manner.

Further, the resetting of the neutral point and the canceling of the reset neutral point are carried out on the bending in both the left and right, and the up and down directions if the angle portion 24 is bent by turning both the LR knob 36 and the UD knob 38.

In this regards, the assistance of bending by the motor is similarly carried out as hereinafter described in detail.

The endoscope 10 of the illustrated example can reset the neutral point with respect to four directions, up and down, and left and right as a preferred embodiment, but this is not the sole case of the present invention.

In other words, in the endoscope of the present invention, the neutral point may be set only in one direction such as only the upward bend and the rightward bend, or may be set in two directions such as only the upward and downward bend, and only the leftward and rightward bend. However, in the endoscope of the present invention, the neutral point is preferably reset for at least the upward bend (so-called up angle). More preferably, the neutral point is reset for the bending in at least both upward and downward directions, and in particular, the neutral point is most preferably reset for the bending in four directions of up and down, and left and right, as in the illustrated example.

In the endoscope 10 of the illustrated example, a motor is used as a driving means for resetting the neutral point of the angle portion 24, but this is not the sole case of the present invention, and various other kinds of driving means may be utilized, as exemplified by solenoids that depend on a fluid pressure or an electromagnetic force to assist in traction of wires.

As described above, the endoscope 10 includes the LR motor 74 for exerting torque on the pulley 68 in correspondence to the bending of the angle portion 24 to the left or the right, the UD motor 92 for exerting torque on the pulley 86 in correspondence to the bending of the angle portion 24 up or down, the torque sensor 100 for sensing the torque exerted on the linking tube 64, and the torque sensor 102 for sensing the torque exerted on the linking tube 84. The manipulation for bending the angle portion 24 may be assisted using the motors 74, 92 and the torque sensors 100, 102.

The assistance of bending the angle portion 24 by the LR motor 74 is described with reference to FIG. 4 using the manipulation of the LR knob 36 for bending the angle portion 24 to the left or the right by way of example.

When the LR motor 74 assists the bending of the angle portion 24, the control means 118 constantly monitors the torque which is exerted on the linking tube 64 and sensed by the torque sensor 100 (i.e., manipulative force exerted on the LR knob 36).

The control means 118 drives the LR motor 74 such that the torque of a predetermined proportion with respect to the torque sensed by the torque sensor 100 is exerted on the pulley 68 and the linking tube 64. As a result, part of the turning force of the LR knob 36 necessary for bending the angle portion 24 is thus assisted by the LR motor 74, and hence the turning of the LR knob 36, namely, the manipulation of bending the angle portion 24 can be performed with a small manipulative force.

For instance, the control means 118 drives the LR motor 74 such that the torque which is exerted on the linking tube 64 and sensed by the torque sensor 100, namely, the torque of constantly equal amount (100%) with respect to the manipulative force of the LR knob 36 is exerted on the pulley 68 and the linking tube 64.

In other words, when torque is exerted on the LR knob 36, the LR motor 74 exerts on the linking tube 64 the torque of equal amount to the torque exerted on the LR knob 36. For instance, if the operator manipulates the LR knob 36 with the force of "50", the LR motor 74 also exerts the same force of "50" on the linking tube 64, whereby the manipulation of bending the angle portion 24 is performed with a total force of "100". In other words, the turning of the LR knob 36 is assisted by the torque of half (50%) of the torque (force) necessary for the bending.

Accordingly, the manipulative force necessary for the operator to bend the angle portion 24 is the manipulative force obtained by subtracting the assisting force from the manipulative force actually required for the target angle, and the LR knob 36 merely needs to be turned with half the torque (manipulative force), and thus the angle portion 24 can be bent with a very small force.

In this case, the LR motor 74 is not limited to assisting with the torque of 100% of the torque exerted on the linking tube 64, and the bending of the angle portion 24 may be assisted in various proportions, as exemplified by such a design that 30% or 80% of the torque that is exerted on the linking tube 64 is supplied by the LR motor 74.

As another assisting method, there is exemplified a method of controlling the assisting force such that the LR motor 74 assists in the bending of the angle portion 24 by supplying an assisting force that is equal to the torque exerted on the linking tube 64 (i.e., manipulative force exerted on the LR knob 36 by the manipulation of the bend) as multiplied by a predetermined coefficient.

Write, for example, W for the force (load) necessary for bending the angle portion 24, F for the torque exerted on the linking tube 64 by the manipulation of the LR knob 36, and T for the assisting force exerted by the LR motor 74 on the linking tube 64. Then, those three forces are related by:

$$W = T + F$$

if the coefficient (gain) of assisting is written as k, the drive of the LR motor 74 is controlled in such a way that T=kF.
In this case,

$$F(1+k) = W$$

$$F = W / (1+k);$$

thus, torque F exerted on the linking tube 64 by the manipulation of the LR knob 36 can accordingly be reduced to be 1/(1+k) times the force that is actually necessary for bending the angle portion 24. If coefficient k is set as 1, the case under consideration is equivalent to the case where 50% of the manipulative force is supplied by the LR motor 74.

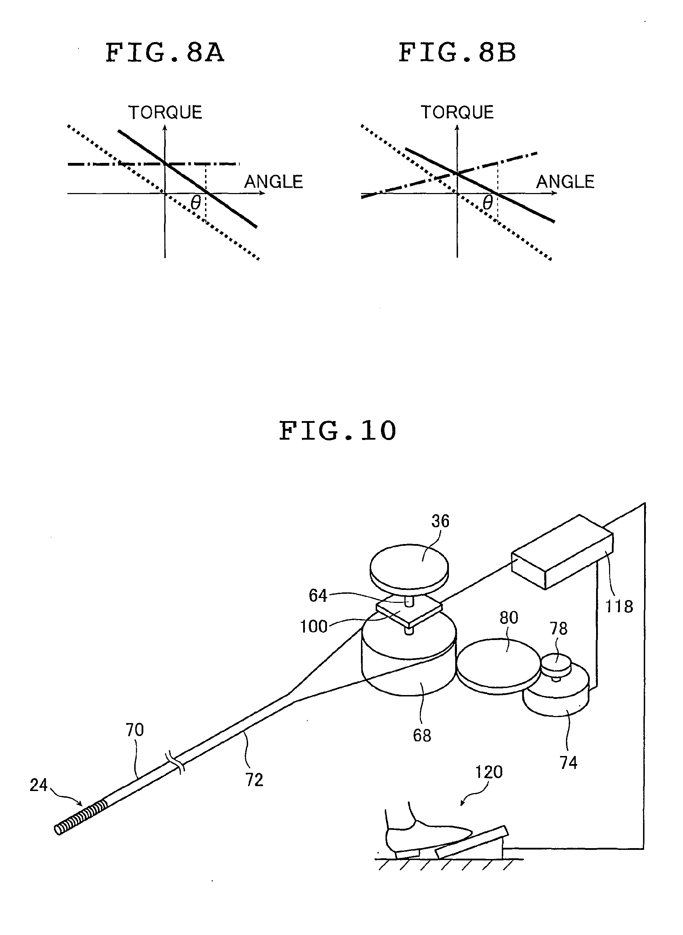

In a state the neutral point of the angle portion 24 is not reset, the relationship of the angle and the torque for bending is as indicated by a dotted line in FIG. 8A. When a constant torque as indicated by a chain dashed line is supplied by the LR motor 74 in order to reset the neutral point to the angle θ, the relationship between the angle and the torque necessary for manipulating the LR knob 36 becomes the state indicated by a solid line in FIG. 8A.

In contrast, when assisting by the LR motor 74 at a predetermined proportion according to the manipulative force of the LR knob 36, the torque by the LR motor 74 becomes tilted diagonally right up with the torque of the LR motor 74 at the angle θ as the center as indicated by a chain dashed line in FIG. 8B when the neutral point is reset at the angle θ. As a result, the relationship between the angle and the torque necessary for manipulating the LR knob 36 becomes the state indicated by a solid line in FIG. 8B, and the manipulative force can be reduced.

Therefore, when the operator performs the basic bending manipulation, and when the traction of the wire 70 or 72, namely, the bending of the angle portion 24 is assisted by the auxiliary means such as the LR motor 74 according to the torque at which the operator turns the LR knob 36 (manipulative force exerted on the manipulation means), the insertion section 12 can be extracted in a safe manner by appropriately turning the LR knob 36 to control the bending of the angle portion 24 even if blackout or a machine trouble such as one in the LR motor 74 occurs. In addition, the bending of the angle portion 24 is basically performed by pulling the wire 70 or 72 with the aid of the manipulation means such as the LR knob 36, and hence the operator can perform the operation of bending the angle portion 24 while feeling the counter force exerting on the angle portion 24 from the part under examination. Thus, an accident such as perforation can be effectively prevented to permit a safe operation for bending the angle portion 24. What is more, the manipulation of the LR knob 36 and other members allows the angle portion 24 to be bent rapidly enough to enable a subtle operation for bending it.

The assisting in bending by the motor 74 is performed in accordance with the torque (manipulative force) applied to the LR knob 36 and other members of the manipulating means. Therefore, if the manipulative force on the LR knob 36 and the like by the operator increases, the pulling of the wires 70 or 72 is assisted in accordance thereto. Thus, even in the case where the manipulative force required by the bending operation is increased due, for example, to aging or deterioration that depends on the situation in which the endoscope has been used such as an increase in the force of friction that develops on the wires 70 and 72 or in the case where, due to the condition of the part under examination, the state of the endoscope at the part under examination or other factors, a great manipulative force is required even for the small amount of bending, the manipulation of the LR knob 36 and other members (force required for their manipulation) can be supplied consistently in accordance with the manipulative force required to bend the angle portion 24.

In this case, in order to ensure that the manipulative force exerted on each of the manipulative knobs for bending the angle portion 24 is detected accurately, the manipulative force exerted on each manipulative knob has to be sensed in a position somewhere between the motor that assists in bending and the manipulative knob (upstream of the motor in the direction in which the manipulative force is transmitted from the manipulative knob toward the assist motor).

In order to meet this need, the manipulating section illustrated in FIGS. 2 and 3 has a torque sensor 100 positioned in the hatched area of the linking tube 64 for sensing the manipulative force (torque) exerted on the LR knob 36, and a torque sensor 102 positioned in the hatched area of the linking tube 84 for sensing the manipulative force exerted on the UD knob 38.

Thus, the illustrated manipulating section 14 adopts such a preferred embodiment in which the torque sensor is positioned at part of the cylindrical linking tube which is directly coupled to a knob for manipulating the angle portion 24 to bend and which rotates integrally with that manipulative knob, whereby the manipulative force exerted on the manipulative knob to bend the angle portion 24 is directly sensed.

When assisting the manipulation of bending by the motor for resetting the neutral point of the angle portion 24, various variations can be utilized other than assisting the manipulative force detected as in the example described above at a constant proportion.

For instance, the manipulative force necessary for bending the angle portion 24 generally becomes larger as the angle (amount of bending) becomes larger. In correspondence thereto, the proportion of the assistance by the LR motor 74 may be increased continuously or in a step-wise manner according to the increase in the manipulative force exerted on the LR knob 36.

In a range where the amount of bending the angle portion 24 is small, the control system tends to easily oscillate with respect to change in the assisting angle, and the angle portion 24 requires a very small amount of force to be bent, which means that the motor has no need to assist in the bending of the angle portion 24. Therefore, if the manipulative force exerted on the LR knob 36 is smaller than or equal to a predetermined value, the LR motor 74 need not assist in the bending of the angle portion 24. In other words, a region that may be called "an insensitive zone" may be provided in the region where only a small amount of manipulative force is applied and no assisting may be performed in such insensitive zone but when a manipulative force beyond the insensitive zone is applied, assisting may be performed in accordance with the applied manipulative force.

Alternatively, a region where the manipulative force applied is smaller than a predetermined value may be assigned as a region where an assisting force is produced in a smaller proportion to the applied manipulative force than in the other regions. For instance, the insensitive zone may be replaced by a low-sensitivity range which is the region where the manipulative force applied is smaller than a predetermined value and where the response (sensitivity) to the applied manipulative force is low; in this low-sensitivity range, the proportion of the assisting force that is produced by the LR motor 74 as relative to the applied manipulative force may be adjusted to be smaller than in the other regions (where the applied manipulative force exceeds the predetermined value).

Conversely, if the manipulative force applied to the LR knob 36 becomes very large, there is high possibility that the angle portion 24 (distal-end portion 22) may have gotten stuck within the human body or may be exerting a strong compressive force within the human body. If, in this case, the angle portion 24 is further forced to bend, an accident such as perforation might even occur to damage the human body. Therefore, if the manipulative force being exerted on the LR knob 36 exceeds a predetermined value, the assisting by the motor 74 may be turned off (suspended).

Alternatively, if the manipulative force being applied to the LR knob 36 exceeds a predetermined value, the assisting force produced by the motor 74 may not be further increased but held constant. In other words, depending on the manipulative force applied to the LR knob 36, a limit may be set on the assisting force that is produced by the motor 74.

The UD motor 92 may assist the bending similarly to the assisting by the LR motor 74.

In the present invention, the embodiments are not limited to being implemented individually when assisting the bending (manipulation) by the motor, and a plurality of embodiments may be combined to assist the bending.

For instance, the embodiment of providing the insensitive zone in the region where only a small amount of manipulative force is applied, and the embodiment of suspending the assistance by the motor in the region where the manipulative force exceeds the predetermined value or the embodiment of setting a limit on the assisting force may be combined.

The embodiment of providing the insensitive zone and the embodiment of providing a low-sensitivity region may be combined so that, with the region up to the first manipulative force being set as the insensitive zone, and the region beyond the first manipulative force up to the second manipulative force being set as the low-sensitivity region, the assisting force of high proportion is applied when exceeding the second manipulative force.

The embodiment of setting a limit on the assisting force and the embodiment of suspending the assisting may be combined so that assisting is carried out with a force of a predetermined proportion corresponding to the manipulative force up to the first manipulative force, the assisting force is set constant with the assisting force in the first manipulative force set as the limit from beyond the first manipulative force up to the second manipulative force, and assisting is not carried out when exceeding the second manipulative force.

In the endoscope of the present invention, the assisting of the bending of the angle portion by the motor for resetting the neutral point may always be performed, or may be performed only when the neutral point is not reset or only when the neutral point is reset. Alternatively, the necessity of assistance may be arbitrarily selected by the operator using an assistance select switch.

Second embodiment

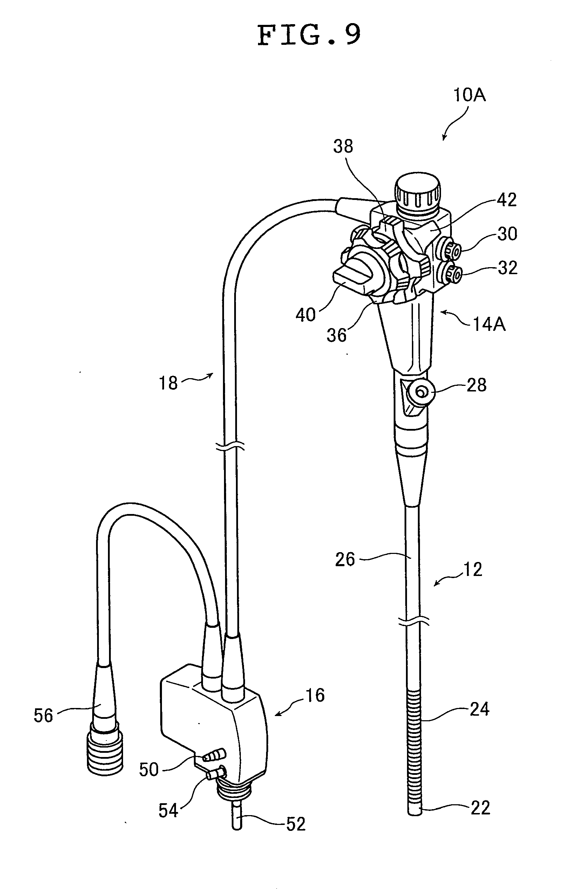

FIG. 9 illustrates an entire structure of an endoscope 10A according to a second embodiment. In the endoscope 10 of the first embodiment, the endoscope 10A, in place of the neutral point resetting function, has a function (hereinafter referred to as pseudo-brake function) of maintaining the manipulation of the manipulative knob immediately before the manipulative force becomes zero, that is, the bent state of the angle portion 24. In the endoscope 10 of the first embodiment illustrated in FIG. 1, the endoscope 10A includes, in place of the manipulating section 14, a manipulating section 14A omitted with the reset switch 44 and the cancel switch 46, and includes, as illustrated in FIG. 10, a pseudo-brake switch 120 connected to the control means 118.

The pseudo-brake switch 120 includes a foot switch, and serves as a selection means for selecting/switching whether or not to apply the pseudo-brake. The pseudo-brake is switched to the pseudo-brake mode ON (apply pseudo-brake) and the pseudo-brake mode OFF (cancel pseudo-brake) every time the pseudo-brake switch 120 is pressed.

The LR motor 74 and the UD motor 92 used as the driving means for resetting the neutral point of the angle portion 24 in the first embodiment are used as a driving means for applying the pseudo-brake for maintaining the angle portion 24 in the bent state in the second embodiment.

In the endoscope 10A, when the manipulative force of the manipulative knob changes to be zero from a value other than zero, the LR motor 74 and the UD motor 92 are driven to bend the angle portion 24 at the manipulative force same as such manipulative force according to the value before the change, whereby the pseudo-brake is applied so as to maintain the angle portion 24 in the bent state without returning to the straight state or the neutral point even when the manipulative force becomes zero, namely, when the operator releases the hand from the manipulative knob.

In other words, when the torque sensed by the torque sensor 100 and the torque sensor 102 changes to be zero from a value other than zero, the LR motor 74 and the UD motor 92 are driven so as to apply such torque on the linking tube 64 and the linking tube 84 according to the value measured immediately before the change. As a result, the pseudo-brake is applied and the manipulative knob is prevented from returning to the neutral point, namely, the angle portion 24 is prevented from returning to the straight state and is maintained in the bent state.

The action of the pseudo-brake is described using the bending of the angle portion to the left or the right in correspondence to the manipulation of the LR knob 36 by way of example. When the pseudo brake switch 120 is switched to the pseudo-brake mode ON, the control means 118 continuously detects the torque measured by the torque sensor 100, namely, the manipulative force for bending exerted on the LR knob 36 at a predetermined timing (sampling rate), and stores the latest sensed torque in the memory.

In addition, the control means 118 reads out from the memory the sensed torque sensed immediately before and stored in the memory when the torque sensed by the torque sensor 100 (i.e., manipulative force of the manipulative knob) becomes zero, and drives the LR motor 74 so that the torque of the same amount as the sensed torque is exerted on the pulley 68 and the linking tube 64.

In other words, as conceptually illustrated in FIG. 11A, at time t0 at which the operator releases the hand from the LR knob 36 and the manipulative force becomes zero, the LR motor 74 is driven such that the torque same as the torque immediately before (manipulative force applied immediately before by the operator) is exerted on the pulley 68 and the linking tube 64, as illustrated in FIG. 11B. In other words, the torque of 100%, with respect to the torque immediately before at which the manipulative force of the LR knob 36 becomes zero, is applied on the pulley 68 and the linking tube 64 by driving the LR motor 74. Therefore, the turning position of the LR knob 36, that is, the bent state of the angle portion 24 when the wires 70 and 72 advance and retreat maintained.

Therefore, even if the operator releases the hand from the LR knob 36 and the manipulative force becomes zero, the LR knob 36 maintains the turning state corresponding to the angle of the angle portion 24, or the angle portion 24 remains in the bent state without returning to the straight state by the counter force as if the brake is applied on the LR knob 36 (pseudo-brake state), similarly to the state immediately before the manipulative force becomes zero.

As apparent from the above description, according to the endoscope 10A of the second embodiment, the operator can safely release the hand from the manipulative knob for bending while bending the angle portion 24, for example, the required manipulation can be carried out with the hand released from the manipulative knob even when performing the manipulation necessary for rotating the insertion section, with the angle portion 24 being greatly bent as in the gastric fundus examination. When further increasing the angle with the angle portion 24 being greatly bent, the operator may once release the hand from the manipulative knob and re-grip the manipulative knob to again manipulate the knob.

Since the half-brake for stopping the turning of the manipulative knob by the frictional force is not applied in this state, the operator can manipulate the bending of the angle portion 24 using the manipulative knob with a normal force without performing the operation of canceling the brake even after the pseudo-brake is applied.

Further, examinations and treatments by the endoscope can be safely performed because the angle portion 24 does not suddenly return to the straight state even if the operator carelessly releases the hand from the manipulative knob, with the angle portion 24 being bent.

In other words, according to the endoscope 10A of the second embodiment, the degree of freedom of various manipulations of the endoscope 10A greatly enhances, the load on the operator who manipulates the endoscope 10A greatly reduces, and the safety of the endoscope further enhances.

In this endoscope 10A, various modes can be utilized for the drive of the LR motor 74 after applying the pseudo-brake (manner of applying the pseudo-brake).

For instance, as illustrated in FIG. 11B, the state in which the pseudo-brake is applied is maintained (output of the LR motor 74 is maintained constant) after applying the pseudo-brake at time t0, and the output of the LR motor 74 is set to zero and the pseudo-brake is canceled when the LR knob 36 is again manipulated at time t1, namely, when the torque sensed by the torque sensor 100 is changed. In this case, the pseudo-brake mode may be automatically switched from ON to OFF (cancel pseudo-brake).

Alternatively, as illustrated in FIG. 11C, once the pseudo-brake is applied at time t0, the state in which the pseudo-brake is applied may be maintained as long a cancel instruction using the provided cancel switch and the like is appropriately issued.

The state in which the pseudo-brake is applied by the LR motor 74 is the state in which the counter force of the angle portion 24 by the bending and the torque exerted by the LR motor 74 are balanced. Therefore, when the LR knob 36 is turned in the direction of further bending the angle portion 24, the angle portion 24 attempts to return to the bent state by the pseudo-brake due to the counter force that increases with increase in angle. Conversely, when the LR knob 36 is turned in the direction of returning the bending of the angle portion 24 by the pseudo-brake, the counter force generates from the torque exerted on the pulley 68 by the LR motor 74, and the angle portion 24 attempts to return to the bent state by the pseudo-brake. In other words, a state similar to when resetting the neutral point of the angle portion 24, which is normally in the straight state, to the bent state is obtained.

The cancel switch for canceling the pseudo-brake may be arranged independently of the pseudo-brake switch 120, or the bent state by the pseudo-brake may be canceled by switching the pseudo-brake mode to OFF.

After applying the pseudo-brake, the LR knob 36 is manipulated so that such manipulation corresponds to the angle smaller than the angle of the angle portion 24 by the pseudo-brake, as indicated by a dotted line in FIG. 11A, namely, the LR knob 36 may be manipulated to bend the angle portion 24 in the direction opposite to the bending direction by the LR motor 74. In this case, in the method illustrated in FIG. 11C, the subsequent pseudo-brake, namely, the torque exerted by the LR motor 74 on the pulley 68 and the linking tube 64 may also be an amount corresponding to the angle in accordance with the manipulation of the LR knob 36, as indicated by a dotted line in FIG. 11C.

Alternatively, a method of maintaining the pseudo-brake and a method of changing the bend by the pseudo-brake according to the manipulation by the LR knob 36 may be selectable with a selection switch and the like.

Further, instead of maintaining the angle by the pseudo-brake constant, the output of the LR motor 74 may be gradually reduced to gradually decrease the angle of the angle portion 24, as illustrated in FIG. 11D after applying the pseudo-brake. In this method, the angle portion 24 ultimately returns to the straight state or the neutral point unless the LR knob 36 is manipulated.

In FIG. 11D, while such pseudo-brake is used for the pseudo-brake illustrated in FIG. 11B, the method of gradually decreasing the angle may be utilized in the pseudo-brake illustrated in FIG. 11C.

Further, two or more methods of the methods illustrated in FIGS. 11B to 11D may be set in advance so that the operator can arbitrarily select the manner of applying the pseudo-brake with the selection switch and the like.

The operator can also choose whether or not to apply the pseudo-brake with the selection switch and the like.

The above description is made using the pseudo-brake corresponding to the bending of the angle portion to the left or the right by the LR knob 36 by way of example. However, but the pseudo-brake is applied in exactly the same manner with respect to the bending of the angle portion up or down by the UD knob 38 at the point the manipulative force becomes zero, namely, at the point the torque sensed by the torque sensor 102 becomes zero.

Further, if the angle portion 24 is bent by both the LR knob 36 and the UD knob 38, the pseudo-brake is applied at the point the manipulative force becomes zero for the bend in both directions.

While in the endoscope 10A of the illustrated example, the motor is used as a driving means for applying the pseudo-brake, this is not the sole case of the present invention. For example, various other kinds of driving means may be utilized, as exemplified by solenoids that depend on a fluid pressure or an electromagnetic force to assist in traction of wires.

In the second embodiment, the pseudo-brake switch 120 is not limited to a foot switch, and may be a selection or switching switch appropriately arranged in the manipulating section 14 of the endoscope 10.

Further, in the endoscope 10, whether the pseudo-brake is turned ON or OFF may be output by displaying on a monitor of the endoscope processor or a display arranged in the manipulating section 14, or audio outputting in the endoscope processor or the manipulating section 14, to enable the operator to easily check the ON/OFF of the pseudo-brake mode.

The endoscope 10 of the illustrated example enables ON/OFF (whether or not to apply pseudo-brake) of the pseudo-brake mode to be selectable as a preferred embodiment. However, this is not the sole case of the present invention, and the pseudo-brake may always be in an applied state.

Similarly to the first embodiment, the manipulation of bending the angle portion 24 can be assisted using the motors 74, 92 and the torque sensors 100, 102 in the second embodiment.

For instance, the control means 118 drives the LR motor 74 such that the torque of half (50%) of the torque applied on the linking tube 64 sensed by the torque sensor 100, namely, the manipulative force of the LR knob 36 is constantly exerted on the pulley 68 and the linking tube 64, as illustrated in FIG. 11E.

In other words, when the torque is applied on the LR knob 36, the LR motor 74 applies the torque of half of such torque on the linking tube 64. For instance, if the operator manipulates the LR knob 36 with a force of "100", the LR motor 74 applies the force of "50", which is half of "100", on the linking tube 64, and thus the angle portion is bent at a force of a total of "150". In other words, the turning of the LR knob 36 is assisted by the torque of 1/3 of the torque (force) necessary for bending the angle portion 24.

Therefore, the manipulative force necessary for the operator to bend the angle portion 24 is the manipulative force obtained by subtracting the assisting force from the manipulative force actually necessary for the target angle, and the LR knob 36 merely needs to be turned at a torque (manipulative force) of 2/3, whereby the angle portion 24 can be bent with a very small force.

The output of the LR motor 74 is changed at the point the manipulative force of the LR knob 36 becomes zero, namely, at the point the torque which is applied on the linking tube 64 and sensed by the torque sensor 100 becomes zero such that the torque similar to the torque sensed by the torque sensor 100 immediately before the point is applied on the linking tube 64.

In this case, the LR motor 74 is not limited to assisting at the torque of 50% of the torque applied on the linking tube 64, and the bending of the angle portion 24 can be assisted at various proportions such as assisting at 30% or 100% of the torque applied on the linking tube 64 with the LR motor 74.

Further, in the endoscope 10A of the second embodiment, the assist of the bending by the motor may be always performed, may be performed only when the pseudo-brake is not applied, or may be performed only when the pseudo-brake is applied; any of which can be selected by the selection switch. Further, the operator may arbitrarily select the necessity of assistance.

Note that the UD motor 92 may assist the bending of the angle portion similarly to that of the LR motor 74.

While the endoscope of the present invention has been described above in detail, the present invention is by no means limited to the first and second embodiments and various improvements and modifications may of course be made without departing from the gist of the present invention.

**Claims**

1. An endoscope comprising an insertion section having a bending portion near its distal end, a state where the bending portion is not bent being set as a neutral point, the endoscope comprising:

   a bending mechanism for bending the bending portion;
   a manipulating section for bending the bending portion through the bending mechanism on the basis of a manipulative force applied by an operator;
   a driving means that applies a driving force on the bending mechanism;
   resetting means that inputs an instruction to reset a state where the bending portion is bent as a new neutral point; and
   control means that controls the driving means so as to maintain the state where the bending portion is bent corresponding to the neutral point reset on the basis of the instruction input by the resetting means.

2. The endoscope according to claim 1, further comprising a manipulative force detection means that detects a manipulative force applied on the manipulating section by the operator, wherein
the control means controls, when an instruction of reset is input by the resetting means, the driving means so as to apply on the bending mechanism a driving force corresponding to the manipulative force detected by the manipulative force detection means.

3. The endoscope according to claim 1, wherein
the bending mechanism comprises a bending member, which is connected to the bending portion, for bending the bending portion by being displaced;
the endoscope further comprises a displacement amount detection means that detects a displacement amount of the bending member corresponding to amount of bending of the bending portion;
the control means comprises a table illustrating a relationship between the displacement amount of the bending member and the driving force of the bending mechanism, the driving force being necessary for bending the bending portion by the amount of bending corresponding to the displacement amount; and
the control means obtains from the table the driving force of the bending mechanism corresponding to the displacement amount detected by the displacement amount detection means and controls the driving means so as to apply the obtained driving force on the bending mechanism when the instruction of reset is input by the resetting means.

4. The endoscope according to claim 1, wherein
the resetting means comprises an instructing means for instructing the amount of bending of the bending portion to be reset as a new neutral point; and
the control means controls, when the instruction of reset is input by the resetting means, the driving means so as to apply on the bending mechanism a driving force necessary for bending the bending portion by the amount of bending instructed by the instructing means.

5. The endoscope according to claim 1, further comprising a manipulative force detection means that detects a manipulative force applied on the manipulating section by the operator, wherein
the control means controls the driving means so as to assist the bending of the bending portion by applying on the bending mechanism a driving force corresponding to a force of a predetermined proportion with respect to the manipulative force detected by the manipulative force detection means.

6. An endoscope comprising an insertion section having a bending portion near its distal end, a state where the bending portion is not bent being set as a neutral point, the endoscope comprising:

a bending mechanism for bending the bending portion;
a manipulating section for bending the bending portion through the bending mechanism on the basis of a manipulative force applied by an operator;
a driving means that applies a driving force on the bending mechanism;
a manipulative force detection means that detects a manipulative force applied on the manipulating section by the operator; and
a control means that controls, when the manipulative force detected by the manipulative force detection means changes to be zero from a value other than zero, the driving means so as to apply to the bending mechanism a driving force corresponding to the value before the change.

7. The endoscope according to claim 6, wherein
the control means controls the driving means so as to assist the bending of the bending portion by applying to the bending mechanism a driving force corresponding to a force of a predetermined proportion with respect to the manipulative force detected by the manipulative force detection means.

8. The endoscope according to claim 6, wherein
the control means controls the driving means so as to stop the application to the bending mechanism of the driving force corresponding to the value when the manipulative force detected by the manipulative force detection means again changes from zero after once changes to be zero.

9. The endoscope according to claim 6, wherein
the control means controls the driving means so as to apply, when the manipulative force detected by the manipulative force detection means changes to be zero, on the bending mechanism the driving force corresponding to the value before the change, and then gradually reduce the driving force.

10. The endoscope according to claim 6, wherein
the control means controls the driving means so as to apply, when the manipulative force detected by the manipulative force detection means changes to be zero, on the bending mechanism the driving force corresponding to the value before the change, and then apply the same driving force until instruction to cancel is issued.

# FIG.1

# FIG.2

# FIG.3

# FIG.4

# FIG.5

## FIG.6A

RESET
NEUTRAL
POINT

$\theta$

## FIG.6B

TORQUE

ANGLE

LOAD OF
ENDOSCOPE

$+$

TORQUE OF MOTOR
FOR RESETTING
NEUTRAL POINT

$=$

$\theta$

TOTAL LOAD

## FIG.6C

TORQUE

$\alpha$  $\alpha$

$\theta$

ANGLE

LOAD
RANGE

RESET
NEUTRAL
POINT

LOAD
RANGE

$\alpha$  $\alpha$

$\theta$

# FIG.7A

DISPLACEMENT OF
ROTATION ANGLE
OF PULLEY OR
DISPLACEMENT OF WIRE

CALCULATE
NECESSARY
TORQUE

DISPLACEMENT-TORQUE
CONVERSION TABLE

# FIG.7B

DISPLACEMENT
SENSOR

# FIG.8A

TORQUE

ANGLE

$\theta$

# FIG.8B

TORQUE

ANGLE

$\theta$

# FIG.10

# FIG.9

## FIG.11A

MANIPULATIVE
FORCE
(TORQUE)

## FIG.11B

100%

TORQUE
APPLIED
BY MOTOR

## FIG.11C

100%

TORQUE
APPLIED
BY MOTOR

## FIG.11D

TORQUE
APPLIED
BY MOTOR

## FIG.11E

100%

50%

TORQUE
APPLIED
BY MOTOR

t0          t1

## FIG.12

Europäisches
Patentamt

European
Patent Office

Office européen
des brevets

# EUROPEAN SEARCH REPORT

Application Number

EP 09 00 3037

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/267093 A1 (MIYAGI TAKAYASU [JP] ET AL) 30 December 2004 (2004-12-30)<br>* abstract *<br>* figures 1-3,5 *<br>* claims 1-20 *<br>* paragraphs [0035], [0050] - [0055], [0063] * | 1,2,4,5 | INV.<br>A61B1/005<br>G02B23/24 |
| Y | | 3 | |
| X | US 2006/069310 A1 (COUVILLON LUCIEN A JR [US]) 30 March 2006 (2006-03-30)<br>* abstract *<br>* figures 1-7 *<br>* paragraphs [0022], [0023], [0025], [0028], [0032], [0037], [0038], [0041], [0042], [0044] - [0052] * | 6-10 | |
| Y | US 5 469 840 A (TANII YOSHIYUKI [JP] ET AL) 28 November 1995 (1995-11-28)<br>* abstract *<br>* figure 10 *<br>* column 1, line 10 - column 1, line 12 * | 3 | |
| | | | TECHNICAL FIELDS SEARCHED (IPC) |
| A | * column 21, line 59 - column 22, line 10 * | 9 | A61B<br>G02B |
| A | US 2004/049097 A1 (MIYAKE KIYOSHI [JP]) 11 March 2004 (2004-03-11)<br>* abstract *<br>* figures 1-18 *<br>* paragraphs [0011] - [0018], [0087], [0113] - [0117], [0128], [0131] - [0133], [0148], [0149], [0155] * | 1-10 | |

-/--

The present search report has been drawn up for all claims

| Place of search | Date of completion of the search | Examiner |
|---|---|---|
| The Hague | 30 June 2009 | Möhrs, Sascha |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 09 00 3037

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 1 884 185 A (OLYMPUS MEDICAL SYSTEMS CORP [JP]) 6 February 2008 (2008-02-06) * abstract * * figures 1-13 * * paragraphs [0001], [0011], [0077], [0112], [0113] * | 1-10 | |
| D,A | JP 2000 229061 A (FUJI PHOTO OPTICAL CO LTD) 22 August 2000 (2000-08-22) * abstract * * figures 1,6 * * paragraph [0001] * | 1-10 | |

**TECHNICAL FIELDS SEARCHED (IPC)**

The present search report has been drawn up for all claims

| Place of search | Date of completion of the search | Examiner |
|---|---|---|
| The Hague | 30 June 2009 | Möhrs, Sascha |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 09 00 3037

The Search Division considers that the present European patent application does not comply with the
requirements of unity of invention and relates to several inventions or groups of inventions, namely:

1. claims: 1-5

   An endoscope comprising means to set and cancel a neutral
   position of a bending portion.
   ---

2. claims: 6-10

   An endoscope comprising force detection and control means to
   automatically detect when a neutral position of the bending
   portion should be set.
   ---

**ANNEX TO THE EUROPEAN SEARCH REPORT
ON EUROPEAN PATENT APPLICATION NO.**                    EP 09 00 3037

This annex lists the patent family members relating to the patent documents cited in the above-mentioned European search report.
The members are as contained in the European Patent Office EDP file on
The European Patent Office is in no way liable for these particulars which are merely given for the purpose of information.

30-06-2009

| Patent document cited in search report | | | Publication date | Patent family member(s) | | | Publication date |
|---|---|---|---|---|---|---|---|
| US 2004267093 | A1 | | 30-12-2004 | JP | 2004321612 | A | 18-11-2004 |
| US 2006069310 | A1 | | 30-03-2006 | US | 2008103362 | A1 | 01-05-2008 |
| | | | | WO | 2006039120 | A1 | 13-04-2006 |
| US 5469840 | A | | 28-11-1995 | NONE | | | |
| US 2004049097 | A1 | | 11-03-2004 | JP | 4169549 | B2 | 22-10-2008 |
| | | | | JP | 2004101737 | A | 02-04-2004 |
| EP 1884185 | A | | 06-02-2008 | CN | 101179980 | A | 14-05-2008 |
| | | | | JP | 2006325745 | A | 07-12-2006 |
| | | | | WO | 2006126550 | A1 | 30-11-2006 |
| | | | | KR | 20070118104 | A | 13-12-2007 |
| | | | | US | 2009048488 | A1 | 19-02-2009 |
| JP 2000229061 | A | | 22-08-2000 | NONE | | | |

EPO FORM P0459

For more details about this annex : see Official Journal of the European Patent Office, No. 12/82

**EP 2 098 159 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 11047082 A
- JP 2000229061 A
- JP 2001346756 A